(19) Europäisches Patentamt
European Patent Office
Office européen des brevets

(11) Numéro de publication: **0 524 108 B1**

(12) # FASCICULE DE BREVET EUROPEEN

(45) Date de publication de fascicule du brevet: **07.06.95**

(51) Int. Cl.[6]: **A61K 7/48**

(21) Numéro de dépôt: **92402077.9**

(22) Date de dépôt: **17.07.92**

(54) **Utilisation de dérivés du benzofuranne dans des compositions cosmétiques ou dermatologiques, comme agents dépigmentants.**

(30) Priorité: **17.07.91 FR 9109028**

(43) Date de publication de la demande:
**20.01.93 Bulletin 93/03**

(45) Mention de la délivrance du brevet:
**07.06.95 Bulletin 95/23**

(84) Etats contractants désignés:
**AT BE CH DE DK ES FR GB GR IT LI NL PT SE**

(56) Documents cités:
**FR-A- 2 285 854**
**GB-A- 2 221 680**

(73) Titulaire: **L'OREAL**
**14, Rue Royale**
**F-75008 Paris (FR)**

(72) Inventeur: **Junino, Alex**
**162, avenue Vauban**
**F-93190 Livry Gargan (FR)**
Inventeur: **N'Guyen, Ouang Lan**
**45, avenue Alsace Lorraine**
**F-92160 Antony (FR)**
Inventeur: **Tuloup, Rémy**

**F-35190 Miniac Sous Becherel (FR)**
Inventeur: **Blaise, Christian**
**24-81, Allée Jan Masaryk**
**F-93270 Sevran (FR)**

(74) Mandataire: **Stalla-Bourdillon, Bernard et al**
**Cabinet Nony**
**29, rue Cambacérès**
**F-75008 Paris (FR)**

**EP 0 524 108 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention a pour objet l'utilisation de dérivés du benzofuranne dans des compositions cosmétiques ou dermatologiques par application topique dans le but de blanchir la peau ou de traiter les taches pigmentaires.

On rappellera que le mécanisme de formation de la pigmentation de la peau, c'est-à-dire de la formation des mélanines est particulièrement complexe et fait intervenir schématiquement les principales étapes suivantes :

Tyrosine ---> Dopa ---> Dopaquinone ---> Dopachrome ---> Mélanines l'enzyme intervenant dans cette suite de réactions étant essentiellement la tyrosinase.

Les substances les plus utilisées à l'heure actuelle en tant que dépigmentants sont plus particulièrement l'hydroquinone et ses dérivés en particulier ses éthers tels que le monométhyléther d'hydroquinone et l'arbutine.

Ces composés, s'ils ont une efficacité certaine, ne sont malheureusement pas exempts d'effets secondaires, ce qui peut rendre leur emploi délicat voir dangereux.

Ainsi l'hydroquinone, dont l'emploi est d'ailleurs limité à une concentration de 2 %, est un composé particulièrement irritant et cytotoxique pour le mélanocyte, dont le remplacement, total ou partiel a été envisagé par de nombreux auteurs.

Il a ainsi été proposé dans le brevet US n° 4 526 179 certains esters gras d'hydroquinone ayant une bonne activité et étant moins irritants et plus stables que l'hydroquinone.

De même on a proposé d'autres dérivés d'hydroquinone dans la demande japonaise n° 27909/86 ne présentant pas les inconvénients de l'hydroquinone mais dont l'efficacité s'est révélée relativement médiocre.

Il est bien établi qu'une substance exerce une action dépigmentante si elle agit directement sur la vitalité des mélanocytes épidermiques où se déroule normalement la mélanogénèse, et/ou si elle interfère avec une des étapes de la biosynthèse des mélanines soit en inhibant un des enzymes impliqués soit en s'intercalant comme analogue structural dans la voie de synthèse qui peut ainsi être bloquée d'où l'effet dépigmentant.

L'utilisation de substances dépigmentantes topiques présentant une bonne efficacité et étant inoffensive, est tout particulièrement recherchée en vue de traiter les hyperpigmentations régionales par hyperactivité mélanocytaire telles que les mélasmas idiopathiques, survenant lors de la grossesse ("masque de grossesse" ou chloasma) ou secondaire à la contraception oestro-progestative, les hyperpigmentations localisées par hyperactivité et prolifération mélanocytaire bénigne telles que les taches pigmentaires séniles dites lentigo actiniques, les hyperpigmentations accidentelles telles que la photosensibilisation et la cicatrisation post-lésionnelle, ainsi que certaines leucodermies telles que le vitiligo ou, à défaut de pouvoir repigmenter la peau lésée, on achève de dépigmenter les zones de peau normale résiduelle pour donner à l'ensemble de la peau une teinte blanche homogène.

Après de nombreuses études sur différentes substances on a constaté de façon tout à fait surprenante que certains dérivés du benzofuranne avaient une action dépigmentante particulièrement prononcée, une très bonne tolérance cutanée et une activité inhibitrice de la mélanogénèse de 1,5 à 2 fois supérieure à celle de l'hydroquinone.

La présente invention a donc pour objet l'utilisation de dérivés du benzofuranne pour la préparation d'une composition cosmétique ou dermatologique ayant une action dépigmentante, lesdits dérivés du benzofuranne répondant à la formule suivante :

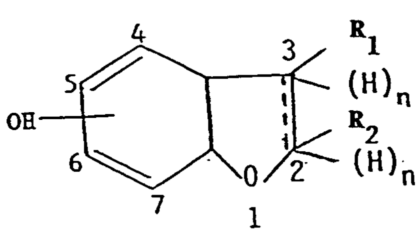

(I)

dans laquelle :

la fonction OH est en position 5 ou 6,

$R_1$ et $R_2$ identiques ou différents, représentent un atome d'hydrogène ou un radical alkyle en $C_1$-$C_4$,

n est 0 ou 1

lorsque n est 0 la liaison $C_2$- $C_3$ est double et
lorsque n est 1 la liaison $C_2$- $C_3$ est simple.

Parmi les composés de formule (I) on peut citer :

le 5-hydroxy-benzofuranne
le 6-hydroxy-benzofuranne,
le 2,3-dihydro 6-hydroxy-benzofuranne,
le 3-méthyl 6-hydroxy-benzofuranne,
le 2,3-diméthyl 6-hydroxy-benzofuranne,
le 2,3-dihydro 3-méthyl 6-hydroxy-benzofuranne, et
le 2-méthyl 5-hydroxy-benzofuranne.

Les dérivés du benzofuranne sont confus et ont été décrits dans la littérature notamment par l'article de René et Royer, Bull. Soc. Chim. Fr, 1973, 2355-2356 et l'article de Oberholzer et al, J.C.S. Perkin I, 1977, 423-426.

Dans les compositions dépigmentantes selon l'invention la concentration en dérivés du benzofuranne de formule (I) est généralement comprise entre 0,01 et 10 % et de préférence entre 0,5 et 5 % en poids par rapport au poids total de la composition.

Le véhicule des compositions peut être notamment une solution aqueuse ou hydroalcoolique, une émulsion du type huile-dans-l'eau ou eau-dans-l'huile, un gel émulsionné ou encore un système biphasé.

De préférence les compositions selon l'invention se présentent sous forme d'une lotion, d'une crème, d'un lait, d'un gel, d'un masque, de microsphères ou nanosphères ou de dispersions vésiculaires. Dans le cas des dispersions vésiculaires, les lipides constitutifs des vésicules peuvent être du type ionique ou non ionique ou bien un mélange de ceux-ci.

Ces compositions cosmétiques peuvent également contenir un humectant, un agent de surface, un kératolytique, un agent anti-inflammatoire, un agent complexant, un anti-oxydant, un conservateur, un parfum ou un filtre solaire.

Les compositions sont appliquées par voie topique en quantité correspondant aux doses d'application usuelles pour le type de composition considéré (gel, crème, lotion, etc...). Par exemple dans le cas d'une crème on utilise de 0,5 à 3 mg et notamment de 1 à 2 mg de crème par $cm^2$ de peau et par application, à raison d'une ou deux applications par jour.

Etudes "in vitro"

Certains des dérivés du benzofuranne ont été étudiés comparativement à l'hydroquinone à quantité molaire équivalente dans le test d'inhibition in vitro de l'activité de la tyrosinase.

Selon ce test on suit par spectrométrie visible à 475 nm la quantité de dopachrome formée au cours de la chaîne de réaction de transformation de la tyrosine en mélanines. Ces réactions sont catalysées in vitro par la tyrosinase de champignons, en présence d'un co-substrat réducteur (par exemple la L-dopa en petite quantité) pour initier la réaction d'hydroxylation de la L-tyrosine en L-dopa, laquelle est ensuite oxydée catalytiquement en dopaquinone puis en dopachrome, produit intermédiaire avant les réactions d'oxydation non enzymatiques aboutissant à la formation des mélanines.

On mesure donc la concentration en dopachrome formé au cours du temps en présence et en absence de l'inhibiteur.

Les concentrations en inhibiteurs sont fixées à 50 % molaire par rapport à la concentration en tyrosine dans le milieu réactionnel.

On exprime l'effet d'inhibition par l'abaissement de la quantité maximale de dopachrome formée (valeur de densité optique à 475 nm lue au plateau de la courbe) par rapport à la quantité obtenue en l'absence d'inhibiteur.

Protocole expérimental

Réactifs :

A - Tampon phosphate 0,1 M pH = 6,5 (Tween 20 à 1 %)
B - Solution mère de L-tyrosine à $2.10^{-3}$M dans A
C - Solution mère de L-dopa à $10^{-4}$ M dans A
D - Solution mère de tyrosinase de champignons à 2.400 unités/ml dans A
E - Solution mère de l'inhibiteur à $10^{-2}$M dans A

(les solutions C et D sont à préparer le jour même)

Résultats

- cuve de référence : 3 ml de A
- cuve d'essai : 1 ml de B
  0,1 ml de C
  1,85ml de A + E
- homogénéiser et équilibrer à 25 °C
- ajouter 0,05 ml de D
- mélanger rapidement et observer la cinétique
  par la mesure de l'absorbance à 475 nm en fonction du temps.

TABLEAU I

| Composés | % inhibition |
|---|---|
| 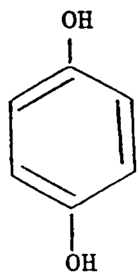<br>hydroquinone | -- 33 % |
| 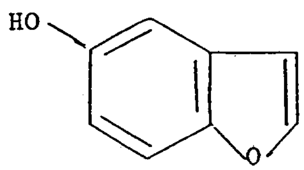<br>5-hydroxybenzofuranne | - 56 % |
| 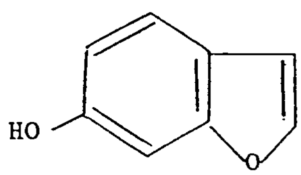<br>6-hydroxybenzofuranne | - 54 % |
| 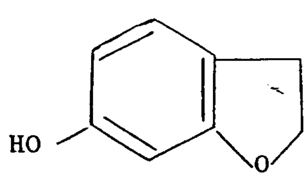<br>2,3-dihydro-6-hydroxybenzofuranne | - 66 % |

Comme on peut le constater les dérivés du benzofuranne des compositions selon l'invention ont une activité inhibitrice de la mélanogénèse nettement supérieure à celle de l'hydroquinone.

EXEMPLE DE PREPARATION

Préparation du 6-hydroxy-benzofuranne

Dans un réacteur équipé d'un réfrigérant, d'un thermomètre, d'une ampoule d'addition, d'une arrivée d'azote et d'un système d'agitation mécanique/chauffage, on introduit 600 $cm^3$ de tétrahydrofuranne (THF).

Après dégazage à l'azote on ajoute, par petites quantités, 14 g de $LiAlH_4$ en 30 minutes. L'addition terminée on porte le mélange réactionnel à 67 ° C. On ajoute alors à cette à cette température, en 2 heures, une solution de 50 g de 6-hydroxy 3-coumaranone dissous dans 1,4 l de THF bouillant.

L'addition terminée, on refroidit à 30 ° C, puis on verse le mélange réactionnel sur 2 l d'une solution HCl 2N sous forte agitation que l'on maintient pendant 30 minutes.

Après extraction par 3 x 300 $cm^3$ d'éther éthylique et lavage de la phase organique par 2 x 200 $cm^3$ d'eau, on sèche sur sulfate de magnésium, filtre puis évapore à sec. On obtient 50 g de produit brut sous forme huileuse.

Le produit brut est ensuite purifié par chromatographie sur colonne de silice dans le dichlorométhane et on obtient 25 g du produit attendu sous forme huileuse. Rdt = 50 %.

EXEMPLES DE COMPOSITIONS

EXEMPLE 1 : Lotion dépigmentante

| | |
|---|---|
| - Alcool éthylique à 96% | 47,8 g |
| - Polyéthylène glycol (8 moles d'OE) | 28,7 g |
| - Ethyl diglycol | 9,5 g |
| - Acétate de sodium trihydraté | 0,06 g |
| - Acide acétique | 0,03 g |
| - Acide N-octanoyl-5-salicylique | 2,0 g |
| - 6-Hydroxybenzofuranne | 2,5 g |
| - Eau | q.s.p. 100,00 g |

Dans cet exemple le 6-hydroxy-benzofuranne peut être remplacé par la même quantité du 2,3-diméthyl 6-hydroxy-benzofuranne ou du 2,3-dihydro 3-méthyl 6-hydroxy-benzofuranne.

EXEMPLE 2 : Emulsion huile-dans-l'eau

| | |
|---|---|
| - Ceteareth 20 (Alcool cétylstéarylique polyoxyéthylénè) | 1,0 g |
| - Palmitostéarate d'éthylène glycol | 3,0 g |
| - Coco caprylate/caprate (esters d'acides en $C_8$-$C_{10}$ et d'alcools gras en $C_{12}$-$C_{18}$) | 5,0 g |
| - Polymère carboxyvinylique vendu sous la dénomination de "CARBOMER 934" par la Société GOODRICH | 0,3 g |
| - Triéthanolamine | 0,9 g |
| - Alcool éthylique à 96 % | 20,0 g |
| - 6-Hydroxybenzofuranne | 1,5 g |
| - Glycérine | 3,0 g |
| - Conservateurs | 0,2 g |
| - Parfum | 0,1 g |
| - Eau | q.s.p. 100,0 g |

Dans cet exemple, le 6-hydroxy-benzofuranne peut être remplacé par la même quantité du 5-hydroxy-benzofuranne ou du 3-méthyl 6-hydroxy-benzofuranne.

EXEMPLE 3 : Emulsion Eau-dans-l'huile

| | |
|---|---|
| - Propylèneglycol | 10,0 g |
| - 6-Hydroxy-benzofuranne | 2,0 g |
| - Huile de vaseline | 20,0 g |
| - Mélange de polycétyldiméthylsiloxane oxyéthyléné oxypropyléné/isostéarate de polyglycéryle à 4 moles de glycérol/laurate d'hexyle | 3,0 g |
| - Conservateurs | 0,2 g |
| - parfum | 0,1 g |
| - Eau | q.s.p. 100,0 g |

Dans cet exemple, le 6-hydroxy-benzofuranne peut être remplacé par la même quantité du 2,3-dihydro 3-méthyl 6-hydroxy-benzofuranne.

EXEMPLE 4 : Emulsion Eau-dans-l'huile

| | |
|---|---|
| - Propylène glycol | 10,0 g |
| - 6-Hydroxy-benzofuranne | 5,0 g |
| - Huile de vaseline | 20,5 g |
| - Esters d'acide gras et de Sorbitol | 5,0 g |
| - Hectorite modifiée par chlorure de stéaryle diméthylbenzylammonium dans le dicaprate de glycéryle | 5,0 g |
| - Coco caprylate/caprate (esters d'acides en $C_8$-$C_{10}$ et d'alcools gras en $C_{12}$-$C_{18}$) | 1,0 g |
| - Conservateurs | 0,2 g |
| - Parfum | 0,1 g |
| - Eau | q.s.p. 100,0 g |

Dans cet exemple le 6-hydroxy-benzofuranne peut être remplacé par la même quantité du 3-méthyl 6-hydroxy-benzofuranne.

## Revendications

**1.** Utilisation de dérivés du benzofuranne pour la préparation d'une composition cosmétique ou dermatologique ayant une action dépigmentante caractérisée par le fait que les dérivés du benzofuranne répondent à la formule suivante :

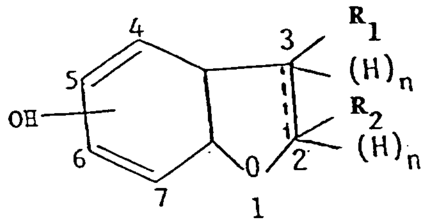

(I)

dans laquelle :
la fonction OH est en position 5 ou 6,
$R_1$ et $R_2$ identiques ou différents, représentent un atome d'hydrogène ou un radical alkyle en $C_1$-$C_4$,
n est 0 ou 1
lorsque n est 0 la liaison $C_2$- $C_3$ est double et
lorsque n est 1 la liaison $C_2$- $C_3$ est simple.

2. Utilisation selon la revendication 1, caractérisée par le fait que les dérivés du benzofuranne sont choisis parmi :
   le 5-hydroxy-benzofuranne
   le 6-hydroxy-benzofuranne,
   le 2,3-dihydro 6-hydroxy-benzofuranne,
   le 3-méthyl 6-hydroxy-benzofuranne,
   le 2,3-diméthyl 6-hydroxy-benzofuranne,
   le 2,3-dihydro 3-méthyl 6-hydroxy-benzofuranne, et
   le 2-méthyl 5-hydroxy-benzofuranne.

3. Utilisation selon l'une quelconque des revendications précédentes caractérisée par le fait que la concentration en dérivés du benzofuranne dans la composition est comprise entre 0,01 et 10 % en poids.

4. Utilisation selon l'une quelconque des revendications précédentes, caractérisée par le fait que la concentration en dérivés du benzofuranne dans la composition est comprise entre 0,5 et 5 % en poids.

5. Utilisation selon l'une quelconque des revendications précédentes caractérisée par le fait que la composition cosmétique ou dermatologique se présente sous forme d'une lotion, d'une crème, d'un lait, d'un gel, d'un masque, de microsphères ou nanosphères ou de dispersions vésiculaires.

6. Utilisation selon l'une quelconque des revendications précédentes, caractérisées par le fait que la composition cosmétique ou dermatologique contient en outre un humectant, un agent de surface, un kératolytique, un agent anti-inflammatoire, un agent complexant, un anti-oxydant, un conservateur, un parfum ou un filtre solaire.

## Claims

1. Use of benzofuran derivatives for the preparation of a cosmetic or dermatological composition having a depigmenting action, characterized in that the benzofuran derivatives correspond to the following formula:

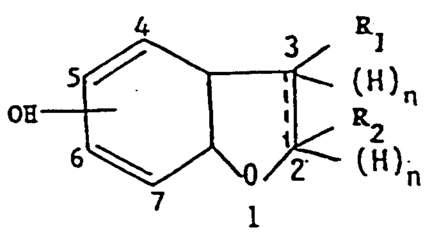

(I)

   in which:
   the OH function is at position 5 or 6,
   $R_1$ and $R_2$, which may be identical or different, represent a hydrogen atom or a $C_1$-$C_4$ alkyl radical,
   n is 0 or 1,
   when n is 0, the C-2 - C-3 bond is double, and
   when n is 1, the C-2 - C-3 bond is single.

2. Use according to Claim 1, characterized in that the benzofuran derivatives are chosen from:
   5-hydroxybenzofuran,
   6-hydroxybenzofuran,
   2,3-dihydro-6-hydroxybenzofuran,
   3-methyl-6-hydroxybenzofuran,
   2,3-dimethyl-6-hydroxybenzofuran,
   2,3-dihydro-3-methyl-6-hydroxybenzofuran, and
   2-methyl-5-hydroxybenzofuran.

3. Use according to either of the preceding claims, characterized in that the concentration of benzofuran derivatives in the composition is between 0.01 and 10% by weight.

4. Use according to any one of the preceding claims, characterized in that the concentration of benzofuran derivatives in the composition is between 0.5 and 5% by weight.

5. Use according to any one of the preceding claims, characterized in that the cosmetic or dermatological composition takes the form of a lotion, cream, milk, gel, mask, microspheres or nanospheres or vesicular dispersions.

6. Use according to any one of the preceding claims, characterized in that the cosmetic or dermatological composition contains, in addition, a humectant, surfactant, keratolytic, anti-inflammatory agent, complexing agent, antioxidant, preservative, perfume or sunscreen agent.

**Patentansprüche**

1. Verwendung von Benzofuranderivaten zur Herstellung einer kosmetischen oder dermatologischen Zubereitung mit Depigmentierungswirkung, dadurch gekennzeichnet, daß die Benzofuranderivate der folgenden Formel entsprechen:

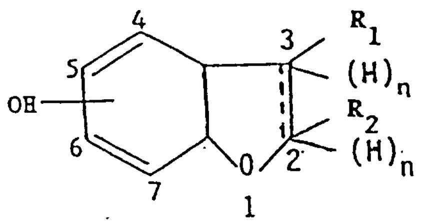

(I)

worin
die funktionelle OH-Gruppe in der 5- oder 6-Stellung steht,
$R_1$ und $R_2$ gleich oder verschieden sind und einen $C_1$-$C_4$-Alkylrest bedeuten,
n = 0 oder 1 bedeutet
und zwar mit der Maßgabe, daß dann,
wenn n = 0 ist, die Bindung $C_2$-$C_3$ eine Doppelbindung darstellt, und dann
wenn n = 1 ist, die Bindung $C_2$-$C_3$ eine Einfachbindung darstellt.

2. Verwendung gemäß Anspruch 1, dadurch gekennzeichnet, daß die Benzofuranderivate unter den folgenden Verbindungen ausgewählt sind:
   5-Hydroxybenzofuran,
   6-Hydroxybenzofuran,
   2,3-Dihydro-6-hydroxybenzofuran,
   3-Methyl-6-hydroxybenzofuran,
   2,3-Dimethyl-6-hydroxybenzofuran,
   2,3-Dihydro-3-methyl-6-hydroxybenzofuran, sowie
   2-Methyl-5-hydroxybenzofuran.

3. Verwendung gemäß einem der vorhergehenden Ansprüche, dadurch gekennzeichnet, daß die Konzentration an Benzofuranderivaten in der Zubereitung zwischen 0,01 und 10 Gew.-% beträgt.

4. Verwendung gemäß einem der vorhergehenden Ansprüche, dadurch gekennzeichnet, daß die Konzentration an Benzofuranderivaten in der Zubereitung zwischen 0,5 und 5 Gew.-% beträgt.

5. Verwendung gemäß einem der vorhergehenden Ansprüche, dadurch gekennzeichnet, daß die kosmetische oder dermatologische Zubereitung in Form einer Lotion, Creme, Milch, eines Gels, einer Maske, in Form von Mikrokügelchen oder Nanokügelchen bzw. vesikulären Dispersionen vorliegt.

**6.** Verwendung gemäß einem der vorhergehenden Ansprüche, dadurch gekennzeichnet, daß die kosmetische oder dermatologische Zubereitung zusätzlich noch ein Feuchthaltemittel, ein Tensid, ein keratolytisches Mittel, ein entzündungshemmendes Mittel, ein Komplexiermittel, ein Antioxidans, ein Konservierungsmittel, einen Duftstoff oder eine Sonnenfilterverbindung enthält.